(19) Europäisches Patentamt
European Patent Office
Office européen des brevets

(11) **EP 2 429 464 B1**

(12) **EUROPÄISCHE PATENTSCHRIFT**

(45) Veröffentlichungstag und Bekanntmachung des Hinweises auf die Patenterteilung:
**27.07.2016   Patentblatt 2016/30**

(21) Anmeldenummer: **10721983.4**

(22) Anmeldetag: **12.05.2010**

(51) Int Cl.:
*A61F 2/91* (2013.01)          *A61F 2/95* (2013.01)

(86) Internationale Anmeldenummer:
**PCT/EP2010/002937**

(87) Internationale Veröffentlichungsnummer:
**WO 2010/130443 (18.11.2010 Gazette 2010/46)**

(54) **MEDIZINISCHE VORRICHTUNG ZUR FREISETZUNG IN EINEM HOHLORGAN UND EINFUHRSYSTEM FÜR MEDIZINISCHE GERÄTE**

MEDICAL DEVICE FOR RELEASING IN A HOLLOW ORGAN AND INSERTION SYSTEM FOR MEDICAL DEVICES

DISPOSITIF MÉDICAL DESTINÉ À ÊTRE LIBÉRÉ DANS UN ORGANE CREUX ET SYSTÈME D'INTRODUCTION POUR APPAREILS MÉDICAUX

(84) Benannte Vertragsstaaten:
**AL AT BE BG CH CY CZ DE DK EE ES FI FR GB GR HR HU IE IS IT LI LT LU LV MC MK MT NL NO PL PT RO SE SI SK SM TR**

(30) Priorität: **13.05.2009   DE 102009021039**

(43) Veröffentlichungstag der Anmeldung:
**21.03.2012   Patentblatt 2012/12**

(73) Patentinhaber: **Acandis GmbH & Co. KG**
**76327 Pfinztal (DE)**

(72) Erfinder:
• **MAILÄNDER, Werner**
**75331 Engelsbrand Grunbach (DE)**

• **CATTANEO, Giorgio**
**76199 Karlsruhe (DE)**

(74) Vertreter: **Kilchert, Jochen**
**Meissner Bolte Patentanwälte**
**Rechtsanwälte Partnerschaft mbB**
**Postfach 86 06 24**
**81633 München (DE)**

(56) Entgegenhaltungen:
**EP-A2- 1 157 673      EP-A2- 1 759 671**
**WO-A1-93/11825      US-A1- 2007 255 386**

**Beschreibung**

Die Erfindung bezieht sich auf eine medizinische Vorrichtung zur Freisetzung in einem Hohlorgan und auf ein Einfuhrsystem für medizinische Geräte.

Eine Vorrichtung zur Freisetzung in einem Hohlorgan mit den Merkmalen des Oberbegriffs des Anspruchs 1 und ein Einfuhrsystem sind beispielsweise aus EP 1 157 673 A2 bekannt, die einen Stent zur transluminalen Implantation in Hohlorganen sowie einen Einführkatheter für einen derartigen Stent offenbart. Zum Implantieren wird der sich im komprimierten Zustand im Einführkatheter befindliche Stent an die zu therapierende Stelle im Hohlorgan gebracht und positioniert. Zum Freisetzen des Stents wird die Außenhülle des Einführkatheters zurückgezogen, so dass der Stent expandiert und das Hohlorgan abstützt.

Für den Fall, dass die Position des aus dem Einführkatheter entlassenen Stents zu korrigieren ist, soll der Stent durch den Einführkatheter repositionierbar sein. Dazu ist der Stent üblicherweise auf einem Führungsdraht des Einführkatheters axial festgelegt, so dass der Stent zusammen mit dem Führungsdraht in den Einführkatheter zurückgezogen werden kann. Bei dem Stentsystem gemäß EP 1 157 673 A2 wird die Repositionierbarkeit des Stents durch ein Positionierelement erreicht, das auf dem Führungsdraht bzw. Mandrin des Einführkatheters befestigt ist. Das Positionierelement weist mehrere auf dem Umfang verteilt angeordnete Ausnehmungen auf, in die entsprechend ausgebildete Halteabschnitte am axialen Stentende formschlüssig eingreifen. Durch die formschlüssige Verbindung zwischen dem Positionierelement und dem Stent wird eine in Axialrichtung wirkende Arretierung geschaffen, die ein Repositionieren des Stents ermöglicht.

Im Bereich der interventionellen Neurologie, insbesondere im Bereich des Stent- und Katheterdesigns geht der Trend der Entwicklung immer weiter in Richtung Miniaturisierung. Soll die vorstehend beschriebene Funktionalität der Repositionierbarkeit in die Konstruktion einfließen, werden schnell die Grenzen der Machbarkeit und der Fertigung erreicht. Dies gilt auch für das aus EP 1 157 673 A2 bekannte Stentsystem, da das Positionierelement aus Festigkeitsgründen im Wandbereich zwischen den Ausnehmungen eine ausreichende Stärke aufweisen muss, die eine Miniaturisierung erschwert bzw. verhindert.

Ein weiteres Stentsystem ist aus US 7,303,580 B2 bekannt, bei dem die Fixierung des Stents auf dem Führungsdraht durch Ösen und diese durchgreifende Nieten erreicht wird, die in das Lumen des Stents hineinragen und einen Anschlag auf dem Führungsdraht hintergreifen. Diese Art der Fixierung lässt sich nicht beliebig verkleinern und hat überdies den Nachteil, dass die Ösen im implantierten Zustand in das Lumen des Stents hineinragen und somit die Blutströmung stören.

Die Druckschrift US 2007/0255386 A1 befasst sich mit einem Stent, der an seinen längsaxialen Enden röntgensichtbare Marker aufweist. Die röntgensichtbaren Marker sind als Coils geformt, die auf einstückig mit der Gitterstruktur des Stents ausgebildeten Halteelementen fixiert sind. EP 1 157 673 A2 beschreibt einen Stent mit einer rohrförmigen Gitterstruktur, wobei an den axialen Enden des Stents Halteelemente ausgebildet sind. Die Halteelemente sind jeweils über Verbindungsabschnitte mit dem axialen Ende der Gitterstruktur verbunden. Die Halteelemente sind im komprimierten Zustand der Gitterstruktur in der Wandungsebene der Gitterstruktur ausgerichtet.

Ein weiterer Stent gemäß dem Stand der Technik ist aus EP 1 759 671 A2 bekannt.

Der Erfindung liegt die Aufgabe zugrunde, eine medizinische Vorrichtung zur Freisetzung in einem Hohlorgan anzugeben, die die Voraussetzung für eine in längsaxialer Richtung wirkende Arretierung in einem Einfuhrsystem zum Positionieren oder Repositionieren der Vorrichtung bildet, wobei sich die Vorrichtung gut für eine kleine Bauweise eignet. Der Erfindung liegt ferner die Aufgabe zugrunde, ein Einfuhrsystem für medizinische Geräte anzugeben, das eine Vorrichtung zur Freisetzung in einem Hohlorgan aufweist, wobei das Einfuhrsystem sich gut miniaturisieren lässt.

Erfindungsgemäß wird die Aufgabe im Hinblick auf die medizinische Vorrichtung durch den Gegenstand des Anspruchs 1 und im Hinblick auf das Einfuhrsystem durch den Gegenstand des Anspruchs 9 gelöst.

Die Erfindung beruht darauf, eine medizinische Vorrichtung zur Freisetzung in einem Hohlorgan mit einem hohlzylindrischen Körper anzugeben, der in einen komprimierten Zustand mit verkleinertem Durchmesser und in einen expandierten Zustand mit vergrößertem Durchmesser überführbar ist, wobei der Körper an seinem proximalen und/oder distalen Axialende ein Arretiermittel aufweist. Das Arretiermittel umfasst wenigstens ein Halteelement, das durch einen Verbindungsabschnitt mit dem Körper verbunden ist. Das Halteelement und der Körper sind durch den Verbindungsabschnitt entkoppelt derart, dass das Halteelement im komprimierten Zustand, insbesondere im komprimierten Zustand des Körpers, bzw. allgemein im komprimierten Zustand der Vorrichtung eine kleinere Krümmung aufweist als der Körper. Demnach ist der Krümmungsradius des Halteelements größer als der Krümmungsradius des Körpers im komprimierten Zustand. Das Halteelement ist weniger stark gekrümmt als der Körper. Der Verbindungsabschnitt verbindet das Halteelement und den Körper elastisch. Die Kontur des Halteelements ragt im komprimierten Zustand des Körpers über den Außenumfang des Körpers hinaus derart, dass das Halteelement durch die Innenwand eines Einfuhrsystems relativ zum Körper radial nach Innen in eine Arretierposition bewegbar ist.

Die Erfindung beruht ferner darauf, ein Einfuhrsystem für medizinische Geräte anzugeben, das eine Vorrichtung zur Freisetzung mit einem Hohlorgan mit einem hohlzylindrischen Körper aufweist, der in einen komprimierten Zustand mit verkleinertem Durchmesser und in einen expandierten Zustand mit vergrößertem Durchmesser überführbar ist, wobei der Körper an seinem proximalen und/oder distalen Axialende ein Arretiermittel aufweist. Das Arretiermittel umfasst wenigstens ein Halteelement, das durch einen Verbindungsabschnitt mit dem Körper elastisch verbunden ist. Das Halteelement und der Körper sind durch den Verbindungsabschnitt entkoppelt derart, dass das Halteelement im komprimierten Zustand, insbesondere im komprimierten Zustand des Körpers, bzw. allgemein im komprimierten Zustand der Vorrichtung eine kleinere Krümmung aufweist als der Körper. Die Kontur des Halteelements berührt die Innenwand des Einfuhrsystems an wenigstens einer Stelle derart, dass das Halteelement relativ zum Körper radial nach Innen in eine Arretierposition bewegt ist und mit einem im Einfuhrsystem angeordneten Element zum Fixieren des Körpers zusammenwirkt.

Dabei ist im komprimierten Zustand der Vorrichtung das Halteelement weniger gekrümmt, also flacher als der Körper. Die Komprimierung der Vorrichtung erfolgt im Wesentlichen, insbesondere vollständig in der Form einer Komprimierung des Körpers. Das Halteelement ist vom Körper entkoppelt, so dass die Komprimierung des Körpers im Wesentlichen zu keiner Formänderung des Halteelements führt.

Im Rahmen der Erfindung wird sowohl die medizinische Vorrichtung zur Freisetzung in einem Hohlorgan an sich, also unabhängig vom Einfuhrsystem, als auch ein Einfuhrsystem mit einer derartigen medizinischen Vorrichtung beansprucht.

Die Erfindung hat den Vorteil, dass die medizinische Vorrichtung einen minimalen Platzbedarf im Einfuhrsystem benötigt. Außerdem lässt sich die Erfindung sehr einfach realisieren, da keine zusätzlichen Positionierelemente erforderlich sind.

Bei einer bevorzugten Ausführungsform der Erfindung sind ein bis vier Halteelemente auf dem Umfang verteilt angeordnet. Dadurch wird erreicht, dass die Halteelemente mit einer ausreichend großen Umfangserstreckung ausgebildet werden können, so dass eine möglichst große nach innen gerichtete Radialbewegung der Halteelemente erreichbar ist. Die Halteelemente können auf dem Umfang im komprimierten Zustand des Körpers beabstandet angeordnet sein. Durch den Freiraum zwischen den Halteelementen im komprimierten Zustand des Körpers wird vermieden, dass die Halteelemente beim Bewegen in die Arretierposition miteinander kollidieren.

Im komprimierten Zustand des Körpers kann die Erstreckung $L_2$ eines Halteelements quer zur Längsrichtung des Körpers wenigstens 5% des Gesamtaußenumfangs des Körpers, insbesondere wenigstens 7%, wenigstens 10%, wenigstens 12%, wenigstens 14%, wenigstens 16%, wenigstens 18%, wenigstens 20%, wenigstens 22%, wenigstens 24%, wenigstens 26%, wenigstens 28%, wenigstens 30% des Gesamtaußenumfangs des Körpers betragen.

Die vorstehend genannten Untergrenzen für die Quererstreckung eines Halteelements haben sich als zweckmäßig erwiesen, um eine ausreichend große Radialverschiebung der Halteelemente nach innen zu erreichen. Die Radialverschiebung nimmt mit zunehmender Größe der Halteelemente zu.

Vorzugsweise beträgt im komprimierten Zustand des Körpers die Erstreckung $L_2$ eines Halteelements quer zur Längsrichtung des Körpers höchstens 32% des Gesamtaußenumfangs des Körpers (10), insbesondere höchstens 30%, höchstens 28%, höchstens 26%, höchstens 24%, höchstens 22%, höchstens 20%, des Gesamtaußenumfangs des Körpers. Damit werden verschiedene Obergrenzen geschaffen, die an eine unterschiedliche Anzahl von Halteelementen angepasst sind.

Ein Halteelement kann im Wesentlichen kreisförmig sein. Die kreisförmige Ausbildung der Halteelemente lässt sich besonders einfach fertigen.

Bei einer weiteren Ausführungsform umfasst das Halteelement einen Röntgenmarker und erfüllt somit eine Doppelfunktion, nämlich einerseits die Arretierfunktion und andererseits die Detektierbarkeit der medizinischen Vorrichtung.

Der Verbindungsabschnitt kann wenigstens einen in Längsrichtung des Körpers angeordneten Steg umfassen. Der Steg bietet eine Möglichkeit, die elastische Verbindung zwischen dem Halteelement und dem Körper herzustellen. Bei dem Einfuhrsystem kann vorgesehen sein, dass das im Einfuhrsystem angeordnete Element einen Führungsdraht umfasst, der wenigstens einen Vorsprung aufweist, wobei das Halteelement den Vorsprung in der Arretierposition hintergreift. Diese Ausführungsform ist besonders einfach zu realisieren, da keine besonderen geometrischen Anforderungen an den Vorsprung gestellt sind.

Bei einer weiteren Ausführungsform weist der Führungsdraht wenigstens zwei Vorsprünge auf, zwischen die das Halteelement in der Arretierposition eingreift. Dadurch wird erreicht, dass das Halteelement und somit die medizinische Vorrichtung in beiden axialen Richtungen fixiert ist, so dass die Vorrichtung in beiden axialen Richtungen durch Betätigen des Führungsdrahtes bewegt werden kann.

Die Erfindung wird nachfolgend anhand von Ausführungsbeispielen unter Bezug auf die beigefügten schematischen Zeichnungen mit weiteren Einzelheiten erläutert. In diesen zeigen:

Fig. 1a    eine perspektivische seitliche Ansicht eines Einfuhrsystems mit einem Stent und einem Führungsdraht, wobei die Halteelemente des Stents sich außerhalb des Einfuhrsystems befinden;

Fig. 1b    eine Ansicht des Systems gemäß Fig. 1a in distaler Richtung;

Fig. 1c    eine Ansicht des Systems gemäß Fig. 1a in proximaler Richtung;

Fig. 2a     das System gemäß Fig. 1a, wobei die Halteelemente in das Einfuhrsystem eingezogen sind;

Fig. 2b     eine Ansicht des Systems gemäß Fig. 2a in distaler Richtung;

Fig. 2c     eine Ansicht des Systems gemäß Fig. 2a in proximaler Richtung;

Fig. 3a     einen schematischen Querschnitt durch ein Einfuhrsystem nach einem weiteren Ausführungsbeispiel, wobei der Schnitt zwischen den Halteelementen und dem Körper verläuft;

Fig. 3b     einen Querschnitt durch das System gemäß Fig. 3a, wobei der Schnitt durch die Halteelemente verläuft;

Fig. 4     einen Längsschnitt durch das System gemäß Figuren 3a, 3b;

Fig. 5     eine schematisierte Draufsicht auf das System gemäß Fig. 4; und

Fig. 6     einen Längsschnitt durch das System gemäß Fig. 4 mit einem Führungsdraht.

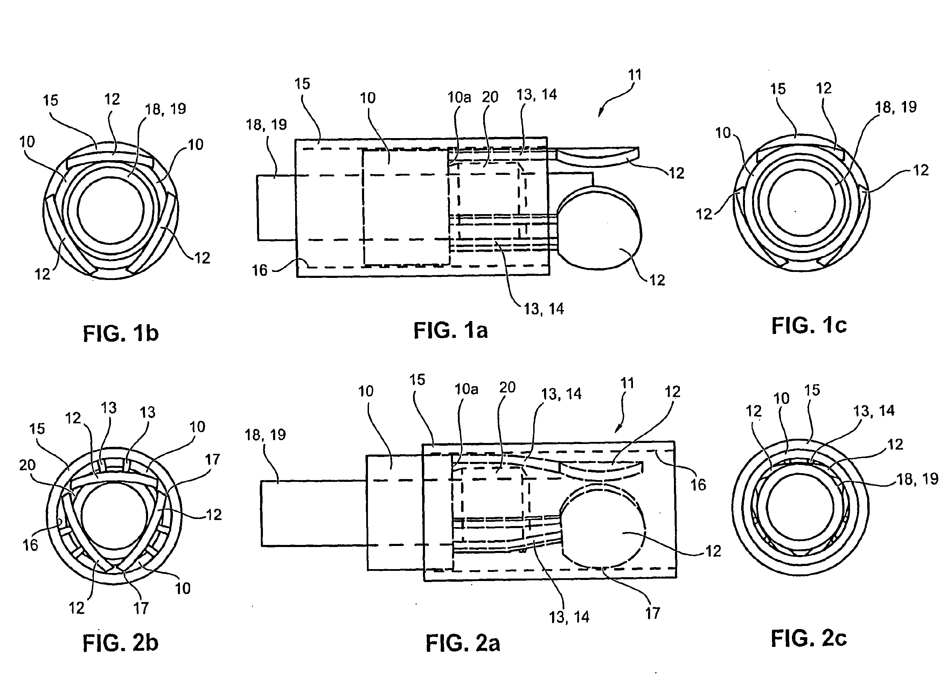

In den Figuren 1a, 1b, 1c ist ein Einfuhrsystem 15 für medizinische Geräte mit einer Vorrichtung zur Freisetzung in einem Hohlorgan dargestellt. Im Rahmen der Erfindung wird Schutz sowohl für die medizinische Vorrichtung an sich, also ohne das Einfuhrsystem, als auch für die Kombination des Einfuhrsystems mit der medizinischen Vorrichtung beansprucht. Die medizinische Vorrichtung kann ein Stent sein, insbesondere für die interventionelle Neurologie. Die Erfindung ist nicht auf Stents eingeschränkt, sondern kann generell für medizinische Implantate verwendet werden, die eine Repositionierung beim Implantieren erforderlich machen. Die Erfindung ist auch auf medizinische Geräte, wie beispielsweise Clot-Retriever, anwendbar, die nicht implantiert, sondern temporär im Körper freigesetzt werden. Damit ist die Erfindung universell im Bereich der Medizintechnik anwendbar und hat aufgrund ihres einfachen Aufbaus ein großes wirtschaftliches Potential.

Alle nachfolgend im Zusammenhang mit dem Stent gemäß Fig. 1a offenbarten Merkmale werden in allgemeiner Form im Zusammenhang mit der medizinischen Vorrichtung zur Freisetzung in einem Hohlorgan offenbart.

Wie in Fig. 1 zu erkennen, weist der Stent einen Körper 10 auf, der ein distales und proximales Axialende hat. In Fig. 1a ist das proximale Axialende 10a dargestellt, das ein Arretiermittel 11 aufweist. Das Arretiermittel 11 kann alternativ am distale Axialende (nicht dargestellt) vorgesehen sein. Es ist auch möglich, dass das Arretiermittel 11 am distalen und proximalen Axialende vorgesehen ist. Das Arretiermittel 11 am distalen und proximalen Axialende kann identisch oder ähnlich ausgeführt sein. Die Ausführungsform mit proximal angeordnetem Arretiermittel 11 ist zum Freisetzen des Stents besonders vorteilhaft, wobei die Erfindung nicht hierauf eingeschränkt ist.

Das Arretiermittel 11 umfasst wenigstens ein Halteelement 12, das durch einen Verbindungsabschnitt 13 mit dem Körper 10 verbunden ist. Zusammen bilden das Halteelement 12 und der Verbindungsabschnitt 13 einen flexiblen Flügel oder eine Zunge, deren Funktion es ist, den Stent im Einfuhrsystem 15 axial festzulegen. Im Ausführungsbeispiel gemäß Fig. 1a sind die Halteelemente 12 jeweils im Wesentlichen kreisförmig oder scheibenförmig ausgebildet. Es ist auch möglich, die Halteelemente 12 beispielsweise oval oder mit anderen Geometrien auszubilden, bspw. in der Form länglicher Zungen, die sich in Richtung des Körpers 10 verjüngen. Im vorliegenden Ausführungsbeispiel sind drei Halteelemente 12 auf dem Umfang verteilt angeordnet. Es ist auch möglich, die Arretierfunktion mit einem einzigen Halteelement 12 oder mit zwei Halteelementen 12 zu erreichen. Es hat sich als zweckmäßig erwiesen, wenn bis zu vier Halteelemente 12 auf dem Umfang verteilt angeordnet sind. Bei einer entsprechenden geometrischen Ausgestaltung der Halteelemente 12 können auch mehr als vier Halteelemente 12 vorgesehen sein, beispielsweise fünf oder sechs Halteelemente 12, vorausgesetzt, die Selbstarretierfunktion der Halteelemente 12 im Zusammenwirken mit dem Einfuhrsystem 15 ist gegeben.

In Fig. 1a ist dargestellt, dass die Halteelemente 12 im expandierten Zustand auf dem Umfang beabstandet angeordnet sind. Durch den Freiraum zwischen den einzelnen Halteelementen 12 wird eine Kollision der Halteelemente 12 bei der Bewegung in die Arretierposition (Fig. 2a) vermieden. Es ist auch möglich, die Halteelemente 12 so zu gestalten, dass diese überlappen und sich in Umfangsrichtung gegeneinander verschieben, so dass eine Kollision der Halteelementkanten beim Komprimieren vermieden wird.

Das Material der Halteelemente 12 kann so beschaffen sein, dass diese als Röntgenmarker dienen. Damit erfüllen die Halteelemente 12 eine Doppelfunktion, nämlich einerseits die mechanische Arretierung des Stents auf dem Führungsdraht 18 und andererseits die Detektierbarkeit des Stents beim Implantieren.

Der Körper 10 bildet eine hohlzylindrische Wandung, die beispielsweise als Closed-Cell-Stentdesign ausgebildet ist. Eine offenzellige Gitterstruktur ist ebenfalls möglich. Die Gitterstruktur kann beispielsweise durch Laserschneiden hergestellt sein. Andere Herstellungsarten, beispielsweise durch Sputtern in Verbindung mit einem Lithografieverfahren oder durch ein Laserabtragsverfahren sind ebenfalls möglich.

Der Verbindungsabschnitt 13 bildet eine elastische Verbindung zwischen dem Halteelement 12 und dem Körper 10. Durch die elastische Verbindung wird erreicht, dass sich die Halteelemente 12 radial nach Innen relativ zum Körper 10 bewegen können, wenn die Halteelemente 12 durch eine Axialbewegung des Körpers 10 mit einer radial nach Innen wirkenden Kraft beaufschlagt werden. Durch die elastische Verbindung wird eine Rückstellkraft erzeugt, die die Halteelemente 12 nach dem Entlassen des Stents aus dem Einfuhrsystem 15 in die aufgeweitete Stellung zurückbewegt. In der aufgeweiteten, expandierten Stellung der Halteelemente 12 erstrecken sich diese in Längsrichtung des Stents und ragen somit nicht in das Stentlumen hinein.

Der Verbindungsabschnitt 13 ist angepasst derart, dass die Halteelemente 12 und der Körper 10 entkoppelt sind. Dazu kann der Verbindungsabschnitt eine kleinere Quererstreckung, d.h. eine kleinere Erstreckung quer zur Längsachse des Stents aufweisen, als die Quererstreckung eines Halteelements 12. Andere Möglichkeiten der Entkopplung sind denkbar, bspw. durch einen geeignete Ausbildung der Gitterstruktur des Körpers 10.

Durch die Entkopplung wird erreicht, dass die Halteelemente 12 im komprimierten Zustand weniger stark gekrümmt sind bzw. einen größeren Krümmungsradius aufweisen als der Körper 10. Das bedeutet, dass die beim Crimpen in das Einfuhrsystem bewirkte Verkleinerung des Stentdurchmessers sich nicht auf die Krümmung bzw. allgemein auf die Geometrie der Halteelemente 12 auswirkt. Die Krümmung der Halteelemente 12 ist damit unabhängig von der Krümmung des Körpers 10 im komprimierten Zustand. Die unterschiedliche Krümmung der Halteelemente 12 und des komprimierten Körpers 10 ist gut in den Figuren 1b, 1c sowie in Fig. 2b zu erkennen.

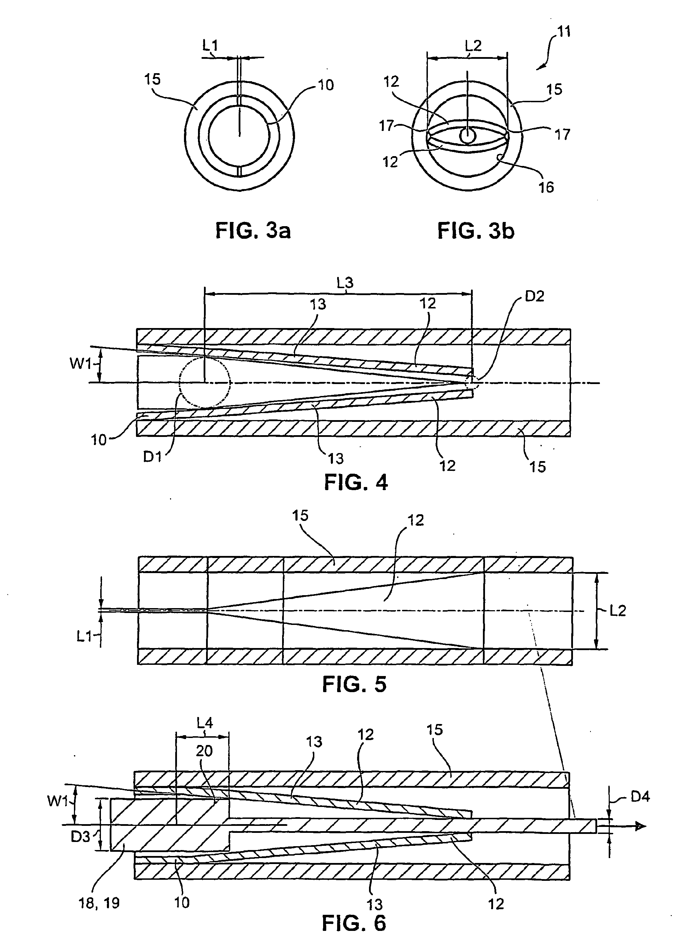

Die Krümmung des Halteelements 12 erfolgt in Umfangsrichtung des Körpers 10. Die Krümmung bzw. der Krümmungsradius des Halteelements 12 ist in den verschiedenen Zuständen (komprimiert, expandiert), die der Körper 10 einnimmt, konstant. Wie in Fig. 3 zu erkennen, sind die in Gegenüberstellung angeordneten Halteelemente 12 in entgegengesetzter Richtung gekrümmt. Die Halteelemente 12 sind dabei jeweils konkav gekrümmt und folgen dabei der Krümmungsrichtung des Körpers 10.

Eine weitere Eigenschaft der Halteelemente 12 besteht darin, dass deren Kontur im komprimierten Zustand des Körpers 10 über dessen Außenumfang und somit über den Innendurchmesser des Einfuhrsystems 15 hinausragt. Dieses Merkmal des Stents ist dadurch überprüfbar, dass der Stent, wie in Fig. 1a dargestellt, im Bereich des Körpers gecrimpt wird und die Halteelemente 12 außerhalb des Einfuhrsystems 15 angeordnet sind. Aufgrund der Entkopplung der Halteelemente 12 vom Körper 10 behalten diese ihre ursprüngliche Krümmung bei. Bei ausreichend großer Umfangserstreckung der Halteelemente 12 ragen diese so weit über den Außenumfang des Körpers 10 bzw. über den Innenradius des Einfuhrsystems 15 hinaus, dass die Halteelemente 12 die Voraussetzung dafür bieten, dass diese bei einer Axialbewegung des Körpers 10 durch die Innenwand 16 des Einfuhrsystems 15 relativ zum Körper 10 radial nach Innen in die Arretierposition bewegt bzw. gedrückt werden können. Die Position der Halteelemente 12 ist in den Figuren 1b, 1c gut zu erkennen, die jeweils zeigen, dass aufgrund des unterschiedlichen Krümmungsradius der Halteelemente 12 und des Körpers 10 sowie aufgrund der Erstreckung der Halteelemente 12 in Umfangsrichtung diese über die Außenkontur des Körpers 10 hinausragen, wenn der Körper 10 komprimiert ist.

Der Verbindungsabschnitt 13 kann beispielsweise durch einen, zwei oder mehr Stege 14 verwirklicht werden, die ein proximales Ende der Halteelemente 12 mit dem distalen Axialende 10a des Stents 10 verbinden. Im Ausführungsbeispiel gemäß Fig. 1a sind zwei Stege 14 vorgesehen. Der Verbindungsabschnitt 13 kann auf andere Weise gestaltet sein, beispielsweise durch einen stärkeren Steg oder durch ein anderes Verbindungsmittel, beispielsweise eine Feder, wenn die in Radialrichtung elastische Verbindung zwischen dem Halteelement 12 und dem Körper 10 gegeben ist.

Es ist auch möglich, dass das Verbindungsmittel bzw. der Verbindungsabschnitt 13 und das Halteelement 12 einteilig ausgebildet sind, wobei das Halteelement 12 profiliert ist und dadurch einen Verbindungsabschnitt bildet. Beispielsweise könnte das Halteelement 12 wie in der Draufsicht gemäß Fig. 5 mit einem im Wesentlichen dreieckförmigen Profil bzw. einer dreieckförmigen Kontur ausgebildet sein, wobei die Spitze der dreieckförmigen Kontur am Körper 10 angelenkt ist. Die Spitze weist dabei eine Breite $L_1$ auf, die kleiner ist, als die Länge $L_2$ einer der Spitze gegenüberliegenden Seite des Dreiecks. Das Dreieck kann beispielsweise ein gleichschenkliges oder ein gleichseitiges Dreieck sein. Das Haltemittel 12 gemäß Fig. 5 kann flächig aus Vollmaterial ausgebildet sein. Alternativ kann die dreiecksförmige Kontur bzw. allgemein die Kontur des Haltemittels 12 durch Stege, insbesondere durch dreieckförmig angeordnete Stege ausgebildet sein.

Das Halteelement 12 kann die Form einer Zunge oder eines Flügels aufweisen, der in Richtung des Körpers 10 sich verjüngt.

Generell gilt, dass die Breite des Verbindungsabschnitts 13 kleiner ist, als die sich quer zur Längsrichtung des Körpers 10 erstreckende Breite des Halteelements 12. Diese Geometrie bezieht sich sowohl auf einfach als auch auf mehrfach ausgebildete Verbindungsabschnitte 13, die bspw. mehrere nebeneinander angeordnete Stege umfassen (Fig. 1a). Bei mehrfach ausgebildeten Verbindungsabschnitten 13 ist die Breite der nebeneinander angeordneten Einzelabschnitte, bspw. der Stege kleiner ist, als die sich quer zur Längsrichtung des Körpers 10 erstreckende Breite des Halteelements 12.

Es ist auch möglich, als Verbindungsmittel bzw. Verbindungsabschnitt 13 die Gitterstruktur im Bereich der axialen Endkante des Körpers 10 einzusetzen, wobei der Übergang zwischen dem Halteelement 12 und der Gitterstruktur so beschaffen ist, dass sich eine Änderung des Krümmungsradius des Körpers 10 beim Crimpen nicht auf den Krümmungsradius des Halteelements 12 überträgt.

Das Verhältnis zwischen der Länge $L_2$ (s. Fig. 5) bzw. der Quererstreckung des Halteelements 12 bezogen auf die Längsachse des Stents zum Innenumfang U der Einführvorrichtung 15, bzw. zum Außenumfang des komprimierten Körpers 10 kann wie folgt berechnet werden: $L_2/U = \sin(180°/n)/\pi$, wobei n = Anzahl der Halteelemente Für vier Halteelemente 12 bzw. Eyelets ergibt sich dabei ein Wert von $L_2/U = 22\%$, für drei Eyelets ein Wert von $L_2/U = 27\%$ und für zwei Eyelets ein Wert von $L_2/U = 32\%$. Diese Werte bilden Obergrenzen für eine entsprechende Anzahl von Halteelementen 12. Es ist möglich, die Halteelemente 12 schmäler zu gestalten, so dass kleinere Werte für das Verhältnis $L_2/U$ erzielbar sind. Die Untergrenze für die Quererstreckung $L_2$ ergibt sich daraus, dass das Querprofil der bzw. eines Halteelements 12 im Ruhezustand über den Außenumfang des komprimierten Körpers 10 hinausragt. Damit wird erreicht, dass durch eine axiale Einschubbewegung des Körpers 10 in das Einfuhrsystem 15 hinein eine auf das Haltelement 12 nach Innen wirkende Radialkraft erzeugt wird, die das Haltelement 12 in die Arretierposition bewegt.

Die maximale Quererstreckung $L_2$ wird durch den Durchmesser des Einfuhrsystems 15 bestimmt, so dass ein Einführen der Halteelemente 12 in das Einfuhrsystem 15 möglich ist.

Wie in Fig. 1a zu erkennen, ist der Abstand $L_3$ (s. Fig. 4) zwischen dem Körper 10 und dem Haltelement 12 größer als die Länge $L_2$ des Halteelements 12 in Querrichtung. Dadurch wird erreicht, dass eine große Bewegungsfreiheit für das Halteelement 12 für die Radialbewegung bereitgestellt wird. Es ist auch möglich, dass der Abstand $L_3$ zwischen dem Körper 10 und dem Haltelement 12 sehr gering ist, so dass das Haltelement 12 praktisch direkt am Körper 10 angeordnet ist. Die Entkopplung des Körpers 10 und des Halteelements 12 zur Beibehaltung eines konstanten Krümmungsradius des Halteelements 12 wird durch einen geeigneten Übergang zwischen dem Halteelement 12 und der Axialkante des Körpers 10 erreicht, bspw. durch eine flexible Gitterstruktur im axialen Randbereich des Körpers 10.

Die Funktionsweise des Stents bzw. allgemein der medizinischen Vorrichtung zur Freisetzung in einem Hohlorgan im Zusammenhang mit dem Einfuhrsystem ist in den Figuren 2a, 2b, 2c sowie in den Figuren 3 bis 6 verdeutlicht.

Das Einfuhrsystem kann beispielsweise einen Einfuhrkatheter bzw. einen Mikrokatheter umfassen und unterliegt keinen besonderen Beschränkungen. Bei dem dargestellten System handelt es sich um ein Rohrsegment beispielsweise eines Mikrokatheters.

Wie der Vergleich der Darstellung gemäß Fig. 1a und Fig. 2a zeigt, wird durch eine axiale Relativbewegung zwischen dem Einfuhrsystem 15 und dem Körper 10 bzw. den Halteelementen 12 die Ortslage der Halteelemente 12 verändert. Konkret werden die Halteelemente 12 durch die Innenwand 16 des Einfuhrsystems 15 radial nach Innen in eine Arretierposition bewegt. In der Arretierposition wirken die Halteelemente 12 mit einem im Einfuhrsystem 15 angeordneten Element 18, beispielsweise mit einem Führungsdraht 19 zum Fixieren des Körpers 10 zusammen. Die Verschiebung der flexiblen Halteelemente 12 wird dadurch erreicht, dass diese eine andere Krümmung als der im Einfuhrsystem 15 befindliche gecrimpte Körper 10 aufweisen. Die Halteelemente 12 berühren die Innenwand 16 an wenigstens einer Stelle 17, insbesondere an zwei Stellen 17, wie in Fig. 2b zu erkennen. Dadurch weichen die Halteelemente 12 radial nach innen aus und werden in die Arretierposition bewegt. Die unterschiedliche Ortslage der Halteelemente 12 ist auch in den Figuren 1b und 2b zu erkennen, die jeweils eine Ansicht des Systems gemäß den Figuren 1a, 2a in distaler Richtung gesehen, zeigen. In Fig. 2b ist zu erkennen, dass die Halteelemente 12 radial weiter innen angeordnet sind, als in der Darstellung gemäß Fig. 1b und einen Vorsprung 20 des Führungsdrahtes 19 hintergreifen, so dass der Körper 10 zumindest in einer axialen Richtung auf dem Führungsdraht 19 festgelegt ist. Der Vorsprung 20 ist in dem vorliegenden Ausführungsbeispiel als ringförmige Schulter ausgebildet, die koaxial zum Führungsdraht 19 angeordnet ist. Andere Geometrien des Führungsdrahtes 19, die als Anschlag für die radial nach innen bewegten Halteelemente 12 dienen, sind möglich.

Die kreisförmige Form der Halteelemente 12 erleichtert das Einführen der Halteelemente 12 in das Einfuhrsystem 15, da die Außenkante des Einfuhrsystems 15 auf dem gekrümmten Rand der Halteelemente 12 abgleiten kann. Andere Profile der Halteelemente 12 sind möglich, insbesondere Profile mit einer im proximalen Bereich der Halteelemente 12 gekrümmten Form. Bei dem Ausführungsbeispiel gemäß den Figuren 1a, 2a bilden die Halteelemente 12 Plättchen, die in radialer Richtung flexibel bewegbar sind. Für die Verbindung der Halteelemente 12 bzw. der Plättchen bieten sich ein oder mehrere Stege 14 an, die die Halteelemente 12 bzw. Plättchen mit dem Körper 10 verbinden. Wie in den Figuren 1a, 2a dargestellt, weisen die Plättchen wenigstens im proximalen bzw. distalen Bereich, bzw. auf der dem Einfuhrsystem zugewandten Seite eine gekrümmte Außenkante auf, die das Einführen der Plättchen in den Mikrokatheter erleichtern.

Im Ausführungsbeispiel gemäß Fig. 2a berühren die Halteelemente 12 die Innenwand 16 an zwei Stellen 17. Es ist auch möglich, das Profil der Halteelemente 12 so zu modifizieren, dass diese die Innenwand 16 an nur einer Stelle oder an mehr als zwei Stellen, beispielsweise an vier, sechs, acht Stellen berühren und so die Radialbewegung der Halteelemente 12 bewirken. Die verschiedenen Stellen, an denen die Innenwand des Einfuhrsystems 15 die Außenkante des Halteelements 12 berührt, können in Längsrichtung des Körpers 12 nachfolgend angeordnet sein. Es ist auch möglich, die Außenkante des Halteelements 12 so zu gestalten, dass ein Linienkontakt zwischen dem Halteelement 12 und der Innenwand 16 zustande kommt. Dabei ist das Halteelement 12 angepasst derart, dass die Berührungskante des Halteelements 12 parallel zur Innenwand 16 verläuft. Im Fall von zwei Berührungskanten verlaufen diese jeweils parallel zur Innenwand 16.

In den Figuren 3 bis 6 ist ein weiteres Ausführungsbeispiel der Erfindung dargestellt, bei dem das Arretiermittel 11 zwei Halteelemente 12 aufweist. Außerdem sind in den Figuren 3 bis 6 die verschiedenen Längen, Durchmesser und Winkel des Arretiersystems im Zusammenhang mit dem Einfuhrsystem beschrieben.

Aus Fig. 3b wird deutlich, dass die Länge $L_2$ eines Halteelements 12 dem maximalen Abstand zwischen den beiden Außenkanten des Halteelements 12 entspricht. Wie in Fig. 3b dargestellt, werden die Halteelemente 12 soweit radial nach Innen bewegt, bis der Abstand $L_2$ dem Innendurchmesser der Innenwand 16 entspricht, wobei das Halteelement 12 die Innenwand 16 an zwei Stellen 17 berührt. Damit kann durch die Breite der Halteelemente 12 konstruktiv festgelegt werden, wie weit die Halteelemente 12 radial nach Innen bewegt werden. Es muss lediglich die Voraussetzung erfüllt sein, dass die Halteelemente 12 breiter sind als der Körper 10 im gecrimpten Zustand.

In Fig. 3a ist dargestellt, dass die Breite $L_1$ des Verbindungsabschnitts 13 am Körper 10 kleiner ist als die Breite $L_2$ eines Halteelements. Damit ist die Anlenkstelle des Flügels am Körper 10 schmäler als der körperferne Bereich des Flügels, so dass einerseits die Entkopplung des Körpers 10 und des Halteelements 12 zur Erzeugung unterschiedlicher Krümmungen und andererseits die flexible Verbindung zwischen dem Körper 10 und dem Halteelement 12 hergestellt ist.

In Fig. 4 ist zu erkennen, dass sich die Verbindungsabschnitte 13 bzw. die Stege 14 verformen, so dass sich ein Winkel $W_1$ über die Länge $L_3$ des Verbindungsabschnittes ergibt. Der Durchmesser $D_2$ zwischen den Halteelementen 12 (s.a. Fig. 3b) ist kleiner als der Durchmesser $D_1$ des Körpers 10. Der Durchmesser $D_1$ des Körpers 10 wird durch Komprimieren des Körpers 10 erreicht. Damit entspricht der Durchmesser $D_1$ des Körpers 10 im Wesentlichen dem Lumen des Mikrokatheters. Der Durchmesser $D_2$ ergibt sich aus dem Durchmesser eines Kreises, der zwischen die gekrümmten Halteelemente 12 einbeschrieben ist, wie in den Figuren 3b und 4 dargestellt. Der Durchmesser $D_2$ entspricht somit dem maximalen Abstand der Innenflächen der in entgegengesetzten Richtungen gekrümmten Halteelemente 12, insbesondere der beiden in entgegengesetzten Richtungen gekrümmten Halteelemente 12.

Das Verhältnis $D_1/D_2$ zwischen dem Durchmesser $D_1$ und dem Durchmesser $D_2$ umfasst folgende Bereiche:

$$D_1/D_2 \geq 1{,}2 \text{ (Untergrenze);}$$

$$D_1/D_2 \leq 10 \text{ (Obergrenze)}$$

Die Untergrenze kann wie folgt variiert werden:

$D_1/D_2 \geq 1{,}4$, insbesondere $\geq 1{,}6$, insbesondere $\geq 1{,}8$, insbesondere $\geq 2$, insbesondere $\geq 2{,}2$, insbesondere $\geq 2{,}4$, insbesondere $\geq 2{,}6$, insbesondere $\geq 2{,}8$, insbesondere $\geq 3$, insbesondere $\geq 3{,}2$, insbesondere $\geq 3{,}4$, insbesondere $\geq 3{,}6$, insbesondere $\geq 3{,}8$, insbesondere $\geq 4$, insbesondere $\geq 4{,}2$, insbesondere $\geq 4{,}4$, insbesondere $\geq 4{,}6$, insbesondere $\geq 4{,}8$, insbesondere $\geq 5$, insbesondere $\geq 5{,}2$, insbesondere $\geq$insbesondere $\geq 5{,}6$, insbesondere $\geq 5{,}8$, insbesondere $\geq 6$, insbesondere $\geq 6{,}2$, insbesondere $\geq 6{,}4$, insbesondere $\geq 6{,}6$, insbesondere $\geq 6{,}8$, insbesondere $\geq 7$, insbesondere $\geq 7{,}2$, insbesondere $\geq 7{,}4$, insbesondere $\geq 7{,}6$, insbesondere $\geq 7{,}8$, insbesondere $\geq 8$, insbesondere $\geq 8{,}2$, insbesondere $\geq 8{,}4$, insbesondere $\geq 8{,}6$, insbesondere $\geq 8{,}8$, insbesondere $\geq 9$, insbesondere $\geq 9{,}2$, insbesondere $\geq 9{,}4$, insbesondere $\geq 9{,}6$, insbesondere $\geq 9{,}8$.

Die Obergrenze kann wie folgt variiert werden:

$D_1/D_2 \leq 9{,}8$, insbesondere $\leq 9{,}8$, insbesondere $\leq 9{,}6$, insbesondere $\leq 9{,}4$, insbesondere $\leq 9{,}2$, insbesondere $\leq 9$, insbesondere $\leq 8{,}8$, insbesondere $\leq 8{,}6$, insbesondere $\leq 8{,}4$, insbesondere $\leq 8{,}2$, insbesondere $\leq 8$, insbesondere $\leq 7{,}8$, insbesondere $\leq 7{,}6$, insbesondere $\leq 7{,}4$, insbesondere $\leq 7{,}2$, insbesondere $\leq 7$, insbesondere $\leq 6{,}8$, insbesondere $\leq 6{,}6$, insbesondere $\leq 6{,}4$, insbesondere $\leq 6{,}2$, insbesondere $\leq 6$, insbesondere $\leq 5{,}8$, insbesondere $\leq 5{,}6$, insbesondere $\leq 5{,}4$, insbesondere $\leq 5{,}2$, insbesondere $\leq 5$, insbesondere $\leq 4{,}8$, insbesondere $\leq 4{,}6$, insbesondere $\leq 4{,}4$, insbesondere $\leq 4{,}2$, insbesondere $\leq 4$, insbesondere $\leq 3{,}8$, insbesondere $\leq 3{,}6$, insbesondere $\leq 3{,}4$, insbesondere $\leq 3.2$, insbesondere $\leq 3$.

Die vorstehend genannten Unter- und Obergrenzen können zur Bildung nach oben und unten begrenzter Bereiche miteinander kombiniert werden.

Ein Beispiel für den größeren Durchmesser $D_1$ beträgt $D_1 = 0{,}7$ mm. $D_1$ kann im Bereich von 0,7 - 0,1 mm variieren, wobei für den Durchmesser $D_1$ alle Zwischenwerte im vorstehend genannten Bereich verwendet werden können.

Beim Herausschieben des Körpers 10 bzw. des Rohrsegmentes wird die Winkeländerung $W_1$ wieder aufgehoben und die Halteelemente 12 kehren wieder in den Ausgangszustand zurück. Damit stellt sich der in Fig. 1a dargestellte Zustand wieder ein. Die Halteelemente 12 legen sich somit an die Gefäßwand an und ermöglichen einen ungehinderten Blutfluss.

In Fig. 5 ist die unterschiedliche Breite der Halteelemente 12 des körperfernen Endes ($L_2$) und des kör-

pernahen Endes (L₁) dargestellt.

Fig. 6 zeigt einen Querschnitt durch ein Einfuhrsystem mit einem Führungsdraht 19, der einen Vorsprung 20 bzw. eine Schulter aufweist, die von den Halteelementen 12 in der Arretierposition hintergriffen werden. Es ist auch möglich, die Durchmesservergrößerung des Führungsdrahtes 19 durch ein Coil auszubilden, der in Längsrichtung fixiert ist. Wird ein Kern bzw. ein Vorsprung 20 mit einem Durchmesser $D_3$, der größer als der Durchmesser $D_4$ ist, in proximaler Richtung verschoben, die durch den Pfeil angedeutet ist, wird das Rohr- bzw. Stentsegment durch den Anschlag zurückgeschoben, der mit dem unter dem Winkel $W_1$ angeordneten Flügel bzw. mit dem dadurch erreichten Hinterschnitt zusammenwirkt. Durch einen weiteren (nicht dargestellten) Anschlag, der distal am Kern bzw. Führungsdraht angebracht ist (nicht dargestellt), kann das Rohrsegment auch in distaler Richtung verschoben werden. Dabei ist der Durchmesser $D_3$ des Kerns bzw. des Vorsprungs 20 etwas kleiner als der Durchmesser $D_1$ und größer als der Durchmesser $D_2$ gemäß Figuren 3b, 4. Dadurch wird der gewünschte Hinterschnitt zur Fixierung des Körpers 10 erreicht. Der Durchmesser $D4_1$ entspricht im Wesentlichen dem Durchmesser $D_2$ zwischen den verengten Halteelementen 12 bzw. ist geringfügig größer als der Durchmesser $D_2$. Die Anordnung zweier Anschläge, die voneinander beabstandet sind derart, dass die Halteelemente 12 in den Freiraum zwischen den beiden Anschlägen eingreifen können, ist für die Anordnung der Arretiermittel 11 am proximalen Axialende des Stents geeignet. Dadurch kann der Stent bzw. allgemein das Rohrsegment in beiden axialen Richtungen, also in proximaler und distaler Richtung, verschoben werden, wodurch die Positionierbarkeit des Stents bzw. des Funktionselements verbessert wird. Im arretierten Zustand kann das Funktionselement in proximaler Richtung in das Einfuhrsystem 15 wiedereingezogen werden (recapturing). Andererseits kann durch Betätigen des Führungsdrahtes in distaler Richtung das Funktionselement aus dem Einfuhrsystem herausgeschoben werden.

Zusammengefasst liegen bei dem Ausführungsbeispiel die Stentenden im Ruhezustand auf derselben Umfangsebene des Stents wie der Körper 10. Die Biegung der Halteelemente 12 radial nach Innen erfolgt durch den Kontakt mit der Katheterinnenwand 16. Der Vorteil besteht dabei darin, dass das nach Innen hineinragende Profil des Stents während der Zufuhr dazu genutzt wird, den Stent in axialer Richtung festzulegen. Im implantierten Zustand legt sich das Profil an die Gefäßwand an, so dass die Gefahr der Thrombenbildung wesentlich reduziert ist.

## Bezugszeichenliste

| | |
|---|---|
| 10 | Körper |
| 10a | Axialende |
| 11 | Arretiermittel |
| 12 | Halteelement |
| 13 | Verbindungsabschnitt |
| 14 | Steg |
| 15 | Einfuhrsystem |
| 16 | Innenwand |
| 17 | Stelle |
| 18 | Element |
| 19 | Führungsdraht |
| 20 | Vorsprung |

## Patentansprüche

1. Medizinische Vorrichtung zur Freisetzung in einem Hohlorgan mit einem hohlzylindrischen Körper (10), wobei die Vorrichtung in einen komprimierten Zustand mit verkleinertem Durchmesser und in einen expandierten Zustand mit vergrößertem Durchmesser überführbar ist und der Körper (10) an seinem proximalen und/oder distalen Axialende (10a) ein Arretiermittel (11) aufweist, das wenigstens ein Halteelement (12) umfasst, welches durch einen Verbindungsabschnitt (13) mit dem Körper (10) verbunden ist,
**dadurch gekennzeichnet, dass**
das Halteelement (12) und der Körper (10) durch den Verbindungsabschnitt (13) entkoppelt sind derart, dass das Halteelement (12) im komprimierten Zustand der Vorrichtung eine kleinere Krümmung aufweist als der Körper (10), und der Verbindungsabschnitt (13) das Halteelement (12) und den Körper (10) elastisch verbindet, wobei die Krümmung des Halteelements (12) in verschiedenen Zuständen, die der Körper (10) einnimmt, konstant ist, und wobei aufgrund der unterschiedlichen Krümmung des Halteelements (12) und des Körpers (10) und aufgrund der Erstreckung des Haltelements (12) in Umfangsrichtung die Kontur des Halteelements (12) im komprimierten Zustand der Vorrichtung über den Außenumfang des Körpers (10) hinausragt derart, dass das Halteelement (12) durch die Innenwand eines Einfuhrsystems relativ zum Körper (10) radial nach Innen in eine Arretierposition bewegbar ist.

2. Vorrichtung nach Anspruch 1,
**dadurch gekennzeichnet, dass**
ein bis vier Halteelemente (12) auf dem Umfang verteilt angeordnet sind.

3. Vorrichtung nach Anspruch 2,
**dadurch gekennzeichnet, dass**
die Halteelemente (12) auf dem Umfang im komprimierten Zustand der Vorrichtung beabstandet angeordnet sind.

4. Vorrichtung nach wenigstens einem der Ansprüche 1 bis 3,

**dadurch gekennzeichnet, dass**

im komprimierten Zustand der Vorrichtung die Erstreckung L2 eines Halteelements (12) quer zur Längsrichtung des Körpers (10) wenigstens 5% des Gesamtaußenumfangs des Körpers (10), insbesondere wenigstens 7%, wenigstens 10%, wenigstens 12%, wenigstens 14%, wenigstens 16%, wenigstens 18%, wenigstens 20%, wenigstens 22%, wenigstens 24%, wenigstens 26%, wenigstens 28%, wenigstens 30% des Gesamtaußenumfangs des Körpers (10) beträgt.

5. Vorrichtung nach wenigstens einem der Ansprüche 1 bis 4,
   **dadurch gekennzeichnet, dass**
   im komprimierten Zustand des der Vorrichtung die Erstreckung L2 eines Halteelements (12) quer zur Längsrichtung des Körpers (10) höchstens 32% des Gesamtaußenumfangs des Körpers (10), insbesondere höchstens 30%, höchstens 28%, höchstens 26%, höchstens 24%, höchstens 22%, höchstens 20%, des Gesamtaußenumfangs des Körpers (10) beträgt.

6. Vorrichtung nach wenigstens einem der Ansprüche 1 bis 5,
   **dadurch gekennzeichnet, dass**
   ein Halteelement (12) mit einem abschnittsweise gekrümmten Umfangsrand, insbesondere im Wesentlichen kreisförmig ausgebildet ist.

7. Vorrichtung nach wenigstens einem der Ansprüche 1 bis 6,
   **dadurch gekennzeichnet, dass**
   das Halteelement (12) einen Röntgenmarker umfasst.

8. Vorrichtung nach wenigstens einem der Ansprüche 1 bis 7,
   **dadurch gekennzeichnet, dass**
   der Verbindungsabschnitt (13) wenigstens einen in Längsrichtung des Körpers (10) angeordneten Steg (14) umfasst.

9. Einfuhrsystem (15) für medizinische Geräte aufweisend eine Vorrichtung nach einem der vorhergehenden Ansprüche, wobei das Halteelement (12) die Innenwand (16) des Einfuhrsystems (15) an wenigstens einer Stelle (17) berührt derart, dass das Halteelement (12) relativ zum Körper (10) radial nach Innen in die Arretierposition bewegt ist und mit einem im Einfuhrsystem (15) angeordneten Element (18) zum Fixieren des Körpers (10) zusammenwirkt.

10. Einfuhrsystem nach Anspruch 9,
    **dadurch gekennzeichnet, dass**
    das im Einfuhrsystem (15) angeordnete Element (18) einen Führungsdraht (19) umfasst, der wenigstens einen Vorsprung (20) aufweist, wobei das Halteelement (12) den Vorsprung (20) in der Arretierposition hintergreift.

11. Einfuhrsystem nach Anspruch 10,
    **dadurch gekennzeichnet, dass**
    der Führungsdraht (19) wenigstens zwei Vorsprünge aufweist, zwischen die das Halteelement (12) in der Arretierposition eingreift.

**Claims**

1. A medical device for releasing in a hollow organ, with a hollow cylindrical body (10), wherein the device can be converted to a compressed state having a reduced diameter and to an expanded state having an enlarged diameter and the body (10) has a locking means (11) at its proximal and/or distal axial end (10a) said locking means comprising at least one retaining element (12) connected to the body (10) by a connecting segment (13),
   **characterized in that**
   the retaining element (12) and the body (10) are decoupled by the connecting segment (13) in such a way that the retaining element (12) has a smaller curvature in the compressed state of the device than the body (10), and the connecting segment (13) elastically connects the retaining element (12) and the body (10), wherein the curvature of the retaining element (12) is constant in different states which the body (10) adopts, and wherein owing to the different curvature of the retaining element (12) and of the body (10) and owing to the extent of the retaining element (12) in circumferential direction, the contour of the retaining element (12) in the compressed state of the device protrudes beyond the outer circumference of the body (10) in such a way that the retaining element (12) can be moved radially inward relative to the body (10) into a locking position by the inner wall of an insertion system.

2. The device according to Claim 1,
   **characterized in that**
   one to four retaining elements (12) are arranged distributed on the circumference.

3. The device according to Claim 2,
   **characterized in that**
   the retaining elements (12) are arranged spaced apart on the circumference in the compressed state of the device.

4. The device according to at least one of Claims 1 to 3,
   **characterized in that**
   in the compressed state of the device the extent L2 of a retaining element (12) transverse to the longitudinal direction of the body (10) measures at least 5% of the total outer circumference of the body (10), in particular at least 7%, at least 10%, at least 12%, at least 14%, at least 16%, at least 18%, at least 20%, at least 22%, at least 24%, at least 26%, at least 28%, at least 30% of the total outer circumference of the body (10).

**5.** The device according to at least one of Claims 1 to 4, **characterized in that**
in the compressed state of the device, the extent L2 of a retaining element (12) transverse to the longitudinal direction of the body (10) measures at most 32% of the total outer circumference of the body (10), in particular at most 30%, at most 28%, at most 26%, at most 24%, at most 22%, at most 20%, of the total outer circumference of the body (10).

**6.** The device according to at least one of Claims 1 to 5, **characterized in that**
a retaining element (12) is designed with a circumferential edge curved in places, in particular in a substantially circular shape.

**7.** The device according to at least one of Claims 1 to 6, **characterized in that**
the retaining element (12) comprises an X-ray marker.

**8.** The device according to at least one of Claims 1 to 7, **characterized in that**
the connecting segment (13) comprises at least one web (14) arranged in the longitudinal direction of the body (10).

**9.** An insertion system (15) for medical devices, having a device according to one of the preceding claims, wherein the retaining element (12) touches the inner wall (16) of the insertion system (15) at at least one location (17), in such a way that the retaining element (12) is moved radially inward relative to the body (10) into the locking position and interacts with an element (18) that is arranged in the insertion system (15) for fixing the body (10).

**10.** The insertion system according to Claim 9, **characterized in that**
the element (18) arranged in the insertion system (15) comprises a guide wire (19) having at least one projection (20), wherein the retaining element (12) engages behind the projection (20) in the locking position.

**11.** The insertion system according to Claim 10, **characterized in that**
the guide wire (19) has at least two projections, between which the retaining element (12) engages in the locking position.

**Revendications**

**1.** Dispositif médical de délivrance dans un organe creux, comportant un corps cylindrique creux (10), le dispositif pouvant être passé dans un état comprimé à diamètre réduit et un état expansé à diamètre accru et le corps (10) présentant à son extrémité axiale proximale et/ou distale (10a) un moyen d'arrêt (11) qui présente au moins un élément de retenue (12) qui est relié par une section de connexion (13) au corps (10),
**caractérisé en ce que**
l'élément de retenue (12) et le corps (10) sont découplés par la section de connexion (13) de manière à ce que l'élément de retenue (12) présente, à l'état comprimé du dispositif, une courbure inférieure à celle du corps (10) et que la section de connexion (13) connecte élastiquement l'élément de retenue (12) et le corps (10), la courbure de l'élément de retenue (12) étant constante dans différents états que le corps (10) prend et, suite à la courbure différente de l'élément de retenue (12) et du corps (10) et suite à l'extension de l'élément de retenue (12) dans le sens circonférentiel, le contour de l'élément de retenue (12) dépassant, à l'état comprimé du dispositif, de la circonférence extérieure du corps (10) de manière à ce que l'élément de retenue (12) soit mobile par la paroi intérieure d'un système d'introduction par rapport au corps (10) radialement vers l'intérieur jusqu'à une position d'arrêt.

**2.** Dispositif selon la revendication 1, **caractérisé en ce qu'**un à quatre éléments de retenue (12) sont disposés répartis sur 1a circonférence.

**3.** Dispositif selon la revendication 2, **caractérisé en ce que**
les éléments de retenue (12) sont disposés espacés sur la circonférence à l'état comprimé du dispositif.

**4.** Dispositif selon au moins une des revendications 1 à 3, **caractérisé en ce que**,
à l'état comprimé du dispositif, l'extension L2 d'un élément de retenue (12) transversalement au sens longitudinal du corps (10) représente au moins 5 % de la circonférence extérieure totale du corps (10), en particulier au moins 7 %, au moins 10 %, au moins 12 %, au moins 14 %, au moins 16 %, au moins 18 %, au moins 20 %, au moins 22 %, au moins 24 %, au moins 26 %, au moins 28 %, au moins 30 % de la circonférence extérieure totale du corps (10).

**5.** Dispositif selon au moins une des revendications 1 à 4, **caractérisé en ce que**,
à l'état comprimé du dispositif, l'extension L2 d'un élément de retenue (12) transversalement au sens longitudinal du corps (10) représente au plus 32 % de la circonférence extérieure totale du corps (10), en particulier au plus 30 %, au plus 28 %, au moins 26 %, au plus 24 %, au plus 22 %, au plus 20 %, de la circonférence extérieure totale du corps (10).

6. Dispositif selon au moins une des revendications 1 à 5,
   **caractérisé en ce**
   **qu'**un élément de retenue (12) est réalisé avec un bord périphérique courbé par endroits, en particulier sensiblement de forme circulaire.

7. Dispositif selon au moins une des revendications 1 à 6,
   **caractérisé en ce que**
   l'élément de retenue (12) comprend un marqueur radiographique.

8. Dispositif selon au moins une des revendications 1 à 7,
   **caractérisé en ce que**
   la section de connexion (13) comprend au moins un longeron (14) disposé dans le sens longitudinal du corps (10).

9. Système d'introduction (15) pour appareils médicaux, présentant un dispositif selon une des revendications précédentes, dans lequel l'élément de retenue (12) touche la paroi intérieure (16) du système d'introduction (15) à au moins un point (17) de manière à ce que l'élément de retenue (12) soit déplacé par rapport au corps (10) radialement vers l'intérieur vers la position d'arrêt et coopère avec un élément (18) disposé dans le système d'introduction (15) pour fixer le corps (10).

10. Système d'introduction selon la revendication 9,
    **caractérisé en ce que**
    l'élément (18) disposé dans le système d'introduction (15) comprend un fil de guidage (19) qui présente au moins une saillie (20), l'élément de retenue (12) étant en prise avec la saillie (20) dans la position d'arrêt.

11. Système d'introduction selon la revendication 10,
    **caractérisé en ce que**
    le fil de guidage (19) présente au moins deux saillies entre lesquelles l'élément de retenue (12) s'engrène dans la position d'arrêt.

FIG. 1b    FIG. 1a    FIG. 1c

FIG. 2b    FIG. 2a    FIG. 2c

EP 2 429 464 B1

FIG. 3a          FIG. 3b

FIG. 4

FIG. 5

FIG. 6

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 1157673 A2
- US 7303580 B2
- US 20070255386 A1
- EP 1759671 A2